# FASCICULE DE BREVET EUROPEEN

(11) **EP 0 721 450 B1**
(45) Date de publication et mention de la délivrance du brevet: **16.12.1998**
(21) Numéro de dépôt: 94930235.0
(22) Date de dépôt: 30.09.1994
(51) Int. Cl.: C07C 303/44, C07C 309/31

(54) **PROCEDE DE TRAITEMENT THERMIQUE ET DE DEGAZAGE DE L'ACIDE SULFONIQUE**
VERFAHREN ZUR WÄRMEBEHANDLUNG UND ZUR ENTGASUNG VON SULFONSÄUREN
METHOD FOR THE HEAT TREATMENT AND DEGASSING OF SULPHONIC ACID

(30) Priorité: 01.10.1993 FR 9311709
(43) Date de publication de la demande: 17.07.1996
(73) Titulaire: Chevron Chemical S.A., F-92527 Neuilly-sur-Seine Cédex (FR)
(72) Inventeur: LE COENT, Jean-Louis, F-76620 Le Havre (FR)
(74) Mandataire: Rinuy, Santarelli
(86) Numéro de dépôt international: FR9401147
(87) Numéro de publication internationale: WO9509840

(56) Documents cités:
- EP-A- 0 002 907
- DE-A- 3 724 036
- FR-A- 1 214 064
- FR-A- 2 067 913

## Description

La présente invention a pour objet un procédé pour éliminer les acides et anhydrides minéraux obtenus lors de la sulfonation d'hydrocarbures lourds, sans production de déchets solides ou liquides.
La fabrication d'acides sulfoniques par la technique du film tombant qui consiste à traiter un hydrocarbure, généralement du type alkylaryl, par de l'anhydride sulfurique est bien connu. La sulfonation se fait dans un tube vertical : l'anhydride sulfurique et l'hydrocarbure (alkylat) sont introduits à co-courant en haut du tube et la sulfonation du film d'alkylat se fait le long des parois.

Elle donne lieu toutefois à la formation d'acides sulfoniques contenant généralement de 0,8 à 3 % en poids d'acides et d'anhydrides minéraux (à savoir essentiellement SO₂ en mélange avec SO₃ et SO₄H₂).

Des teneurs aussi élevées en impuretés minérales acides donnent lieu à des dépôts importants de sédiments de sulfate de calcium, toujours difficiles à filtrer, lors de la neutralisation de l'acide sulfonique par de la chaux, pour obtenir du sulfonate de calcium, utilisé comme détergent dans l'industrie des additifs pour lubrifiant.

Pour y remédier, la demande de brevet européen 2.907 d'EXXON propose un lavage à l'eau, un traitement avec une oléfine et un traitement thermique final.

Ce procédé antérieur, s'il permet effectivement de réduire la teneur en acide sulfurique résiduel de l'acide sulfonique ainsi traité, par réaction des impuretés minérales avec une oléfine, présente toutefois de sérieux problèmes de corrosion, qui limitent sa mise en oeuvre, malgré l'utilisation d'un appareillage en acier inoxydable.

La présente invention se propose de réduire la teneur en impuretés minérales du type SO₂, SO₃, SO₄H₂ d'un acide sulfonique, non pas en faisant réagir les impuretés avec une oléfine, comme dans la demande de brevet européen précitée, mais par dégazage, impliquant un traitement thermique sous agitation avec barbotage d'azote.

Selon l'invention, on chauffe l'acide sulfonique à une température comprise entre 70 et 100°C, notamment entre 75 et 95°C, de préférence vers 85°C, sous agitation et barbotage d'azote, éventuellement après dilution de l'acide par environ 5 à 20 % en poids et notamment 10 % d'huile pour diminuer la viscosité et faciliter l'élimination des anhydrides sulfureux et sulfuriques par dégazage.

Ce traitement thermique est poursuivi pendant la durée nécessaire pour obtenir une teneur au moins inférieure à 0,5 et de préférence de l'ordre de 0,3 % en poids d'acide sulfurique ; cette durée s'est avérée être de l'ordre de 1 à 3 jours pour une teneur de 0,3 % en poids de SO₄H₂.

On peut également opérer sous vide pour diminuer la durée du traitement thermique : c'est ainsi qu'à une température de 85°C sous agitation et barbotage d'azote, on peut réduire la durée du traitement thermique de 24 heures à 12 heures en opérant sous un vide de 6,7 KPa (50 mm Hg) de pression absolue.

Des températures inférieures à 70°C ne permettent pas d'obtenir une élimination des impuretés minérales acides dans un temps raisonnable sur le plan industriel.

Et des températures supérieures à 100°C, si elles permettent de réduire la durée du traitement thermique à une durée de l'ordre de 1 H 30 environ, pour une température de 140°C, posent toutefois des problèmes de corrosion, qui nécessitent l'utilisation d'un appareillage spécial, tel qu'un réacteur et des conduites vitrifiées.

Il convient de noter que la teneur optimum résiduelle en acide sulfurique de l'acide sulfonique est de l'ordre de 0,30 % en poids, car au-dessus de cette teneur le risque de dépôt augmente et au-dessous la stabilité thermique de l'acide sulfonique diminue et il a tendance à se décomposer.

La teneur en acide sulfurique est déterminée par potentiométrie et l'analyse comprend, après addition d'eau, une mesure de l'acidité de l'acide sulfonique et de la première acidité de l'acide sulfurique et une mesure de la seconde acidité de l'acide sulfurique.

Les acides sulfoniques auxquels s'applique le procédé selon l'invention répondent à la formule générale : 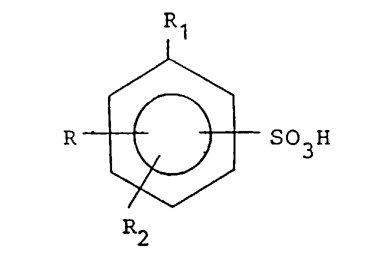 où R1 et R2 représentent de l'hydrogène ou des groupes hydrocarbures et notamment des groupes alkyles tels que méthyle ou éthyle et où R est un groupe alkyle contenant de 12 à 40 atomes de carbone, qui peut être linéaire ou ramifié.

Les acides alkylaryl sulfoniques préférés, dans le procédé selon l'invention, sont les acides mono- ou polyalkyl phényl sulfoniques, dans lesquels le ou les groupes alkyl en C₁₂ à C₄₀ proviennent au moins à 80% et de préférence à 100% en poids de la polymérisation de l'éthylène et sont donc des groupes alkyles au moins à 80% et de préférence à 100% en poids linéaires.

Ces acides alkylaryl sulfoniques proviennent de composés aromatiques alkylés, qui sont généralement connus dans le commerce sous le nom d'alkylats.

Parmi les huiles de dilution pouvant être mises en oeuvre selon l'invention, on peut citer de préférence les huiles paraffiniques telles que l'huile 100 Neutral ; les huiles naphténiques ou mixtes peuvent également convenir.

Ces huiles de dilution sont choisies de préférence de façon à présenter une viscosité à la température de 100°C de l'ordre de 3 à 5.10⁻⁶ m²/s. (3 à 5 centistokes).

### EXEMPLE 1

Dans un réacteur en verre de 4 litres, on introduit successivement :
- 2 kg d'acide sulfonique de masse moléculaire 458 provenant de la sulfonation d'un mélange d'alkylat linéaire/ramifié commercialement appelé AL 584 (80% linéaire et 20% ramifié).
- 200 g d'huile 100 Neutral.

Le mélange est chauffé à 85°C sous agitation.

A cette température, un barbotage à l'azote est effectué à une débit de 10 l/h.

La durée du traitement est de 30 heures.

Les teneurs en % en poids en ions HSO₃⁻ provenant de l'acide sulfonique et en H₂SO₄ des différents échantillons analysés par potentiométrie sont indiqués dans le tableau 1 ci-dessous.

**TABLEAU 1**

| | % HSO₃⁻ | % H₂SO₄ | % Sédiment |
|---|---|---|---|
| Au chargement avant dilution | 14,1 | 0,95 | |
| Au chargement après dilution par huile | 12,93 | 0,73 | 0,004 |
| Après traitement | 12,92 | 0,16 | 0,004 |

### EXEMPLE 2

Cet exemple est identique à l'exemple ci-dessus, sauf que le traitement est effectué pendant 12 heures sous un vide de 6,7 KPa (50mm Hg) de pression absolue. Un résultat pratiquement identique est obtenu après le traitement % HSO₃⁻ = 12,92, % H₂SO₄ = 0,14, % Sédiments : 0,004

### EXEMPLE 3

Dans un réacteur en verre de 4 litres, on introduit successivement :
- 2923,6 g d'acide sulfonique d'alkylat linéaire de masse moléculaire 470 (100% linéaire, le groupe alkyle ayant été obtenu exclusivement par polymérisation de l'éthylène).
- 292,4 g d'huile 100 Neutral.

Les conditions de traitement sont :
- température 85°C à pression atmosphérique
- agitation,
- débit de l'azote : 10 l/heure.

Plusieurs échantillons sont prélevés au cours du temps et les analyses sont récapitulées dans le tableau 2 suivant où le temps t = 0 correspond, après addition de l'huile 100 N, à l'obtention d'une température de 85°C.

**TABLEAU 2**

| TEMPS EN HEURES | % HSO₃⁻ en poids | % H₂SO₄ en poids |
|---|---|---|
| 0 | 14,65 | 1,3 |
| 5 | 14,91 | 0,84 |
| 22 | 14,9 | 0,62 |
| 29 | 14,9 | 0,54 |
| 36 | 14,7 | 0,42 |
| 52 | 14,8 | 0,37 |
| 59 | 14,9 | 0,20 |

### EXEMPLES 4, 5 ET 6

L'influence de la température sur la durée de traitement est testée dans ces trois essais.

Un même acide est utilisé et dilué avec 10 % d'huile 100 N. L'analyse de la solution est % HSO₃⁻ = 11,92, % H₂SO₄ = 1,96.

Cette dernière est ajoutée dans les 3 réacteurs identiques agités avec un barbotage à l'azote de 10 l/h. Les résultats analytiques concernant les teneurs en % HSO₃⁻ et % H₂SO₄ figurent dans le tableau 3 ci-dessous.

Ce tableau met clairement en évidence l'influence de la température : sur un produit ayant de l'ordre de 2 % de H₂SO₄ résiduaire, le temps nécessaire pour une réduction satisfaisante de la teneur en H₂SO₄ est de 72 h à 85°C, 48 h à 90°C et 24 h à 100°C.

**TABLEAU 3**

| Température en °C | Temps en heures | % HSO₃⁻ en poids | % H₂SO₄ en poids |
|---|---|---|---|
| 85 | 24 | 11,92 | 0,80 |
| | 48 | 12,12 | 0,54 |
| | 72 | 12,15 | 0,35 |
| 90 | 24 | 12,19 | 0,60 |
| | 48 | 12,07 | 0,30 |
| 100 | 24 | 12,15 | 0,25 |

## Revendications

1. Procédé de réduction des impuretés minérales du type SO₂, SO₃ et SO₄H₂ dans un acide sulfonique, caractérisé en ce qu'il consiste à soumettre cet acide sulfonique à un traitement thermique à une température comprise entre 70 et 100°C, sous agitation et avec barbotage d'azote, pendant un temps suffisant pour obtenir une teneur en acide sulfurique résiduel au moins inférieure à 0,5 % en poids et de préférence de l'ordre de 0,3 % en poids.

2. Procédé selon la revendication 1, caractérisé en ce que le traitement thermique a lieu à une température comprise entre 75 et 95°C, sous agitation et avec barbotage d'azote, pendant une période de l'ordre de 1 à 3 jours, jusqu'à l'obtention d'une teneur en acide sulfurique résiduel de 0,3 % en poids.

3. Procédé selon la revendication 2, caractérisé en ce que la température de traitement thermique est de 85°C.

4. Procédé selon l'une quelconque des revendications précédentes, caractérisé en ce que le traitement thermique est effectué sous vide.

5. Procédé selon l'une quelconque des revendications précédentes, caractérisé en ce que le traitement thermique est effectué en présence de 5 à 20 % et de préférence 10 % en poids d'huile.

6. Procédé selon l'une quelconque de revendications précédentes, caractérisé en ce que l'acide sulfonique est un acide alkylphénylsulfonique dans lequel le ou les groupes alkyles en C₁₂ à C₄₀ proviennent au moins à 80% et de préférence à 100 % en poids de la polymérisation de l'éthylène.

## Patentansprüche

1. Verfahren zur Verringerung der anorganischen Verunreinigungen des Typs SO₂, SO₃ und SO₄H₂ in Sulfonsäuren, dadurch gekennzeichnet, dass das Verfahren umfasst das Unterwerfen der Sulfonsäure einer Wärmebehandlung bei einer Temperatur zwischen 70 und 100°C, unter Rühren und Durchleiten von Stickstoff über einen Zeitraum, dass ein Rest-Schwefelsäuregehalt von mindestens weniger als 0,5 Gew.% erhalten wird, vorzugsweise im Bereich von 0,3 Gew.%.

2. Verfahren nach Anspruch 1, wobei die Wärmebehandlung bei einer Temperatur zwischen 75 und 95°C erfolgt, unter Rühren und Durchleiten von Stickstoff über einen Zeitraum von 1 bis 3 Tagen bis ein Rest-Schwefelsäuregehalt von 0,3 Gew.% erreicht ist.

3. Verfahren nach Anspruch 2, wobei die Wärmebehandlung bei.einer Temperatur von 85°C erfolgt.

4. Verfahren nach einem der vorstehenden Ansprüche, wobei die Wärmebehandlung unter Vakuum erfolgt.

5. Verfahren nach einem der vorstehenden Ansprüche, wobei die Wärmebehandlung in Gegenwart von 5 bis 20 Gew.%, vorzugsweise 10 Gew.% Öl erfolgt.

6. Verfahren nach einem der vorstehenden Ansprüche, dadurch gekennzeichnet, dass die Sulfonsäure eine Alkylphenylsulfonsäure ist, bei der die C₁₂-C₄₀-Alkylgruppe oder -gruppen wenigstens zu 80 Gew.%, vorzugsweise zu 100 Gew.% aus der Polymerisation von Ethylen stammen.

## Claims

1. Process for reducing mineral impurities of SO₂, SO₃ and SO₄H₂ type in a sulphonic acid, characterized in that it consists of subjecting this sulphonic acid to a thermal treatment at a temperature comprised between 70 and 100°C, accompanied by stirring and with nitrogen bubbling, for a time sufficient to obtain a level of residual sulphuric acid of at least less than 0.5% by weight and preferably of the order of 0.3% by weight.

2. Process according to claim 1, characterized in that the thermal treatment takes place at a temperature comprised between 75 and 95°C, accompanied by stirring and with nitrogen bubbling, for a period of time of the order of 1 to 3 days, until a level of residual sulphuric acid of 0.3% by weight is obtained.

3. Process according to claim 2, characterized in that the thermal treatment temperature is 85°C.

4. Process according to any one of the preceding claims, characterized in that the thermal treatment is carried out under vacuum.

5. Process according to any one of the preceding claims, characterized in that the thermal treatment is carried out in the presence of 5 to 20% and preferably 10% by weight of oil.

6. Process according to any one of the preceding claims, characterized in that the sulphonic acid is an alkylphenylsulphonic acid in which at least 80% and preferably 100% by weight of the C₁₂ to C₄₀ alkyl group or groups originate from the polymerization of ethylene.
